(19) Europäisches Patentamt
European Patent Office
Office européen des brevets

(11) EP 4 678 623 A1

(12) EUROPEAN PATENT APPLICATION

(43) Date of publication:
14.01.2026 Bulletin 2026/03

(21) Application number: 24188452.7

(22) Date of filing: 12.07.2024

(51) International Patent Classification (IPC):
*C07C 43/04* (2006.01) *C07C 41/01* (2006.01)

(52) Cooperative Patent Classification (CPC):
(C-Sets available)
C07C 41/01; C07C 41/58; C07C 43/043 (Cont.)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
GE KH MA MD TN

(71) Applicant: **Nederlandse Organisatie voor toegepast-natuurwetenschappelijk onderzoek TNO**
**2595 DA 's-Gravenhage (NL)**

(72) Inventor: **The designation of the inventor has not yet been filed**

(74) Representative: **Nederlandsch Octrooibureau**
**P.O. Box 29720**
**2502 LS The Hague (NL)**

# (54) PROCESS FOR PRODUCING DIMETHYL ETHER

(57) The present invention concerns a process for the synthesis of dimethyl ether (DME) from a feedstock comprising sub-stoichiometric amounts of $H_2$, which was found to reduce the formation of the undesirable by-product MeOH. The feedstock comprises $H_2$ and $CO_x$, wherein x in the range of 1.0 - 2.0 and the stoichiometry factor M in the range of 0.05 - 1.7, wherein M is defined as:

$$M = \frac{[H_2] - [CO_2]}{[CO] + [CO_2]}$$

The process of the invention includes a step (a) wherein the feedstock is subjected to separation-enhanced DME synthesis by contacting it with one or more catalysts together capable of converting synthesis gas to DME, to obtain a product mixture comprising DME.

EP 4 678 623 A1

(52) Cooperative Patent Classification (CPC): (Cont.)

C-Sets
**C07C 41/01, C07C 43/043;**
**C07C 41/58, C07C 43/043**

## Description

### Field of the invention

The present invention relates to the production of dimethyl ether (DME) from a feedstock comprising $H_2$ and $CO_x$.

### Background

Dimethyl ether (DME) is a valuable compound useful in the chemical industry, e.g. as precursor of dimethyl sulphate, acetic acid or for olefin production. It is an important research chemical and is used as refrigerant and propellant. Moreover, DME may find more widespread use in the future, as it is being developed as novel fuel, e.g. as replacement for or additive to propane in LPG and as diesel fuel additive. It can be produced by catalytic dehydration of methanol, and the methanol can be produced by catalytic hydrogenation of CO or $CO_2$, e.g. using synthesis gas. The production of DME from synthesis gas may thus be accomplished via the direct or the indirect method. The indirect method involves contacting the synthesis gas with a methanol synthesis catalyst to form methanol, which is subsequently contacted with a dehydration catalyst to form DME. Alternatively, methanol could be used as a starting material which is contacted with a dehydration catalyst without the need of a methanol synthesis catalyst. The direct method involves contacting the synthesis gas with a bifunctional catalyst comprising a methanol synthesis catalyst and a dehydration catalyst, such that isolation and purification of the methanol is not required. Both the direct and the indirect method are presently commercially used for the production of DME.

Several reaction schemes for the synthesis of dimethyl ether have been developed (see e.g. Dieterich et al., Energy Environ. Sci., 2020, 13, 3207-3252; see section 3.1), such as:

| | |
|---|---|
| (1) Methanol synthesis: | $CO_2 + 3\ H_2 \leftrightarrow CH_3OH + H_2O$ |
| (2) Water gas shift: | $CO + H_2O \leftrightarrow H_2 + CO_2$ |
| (3) Methanol dehydration: | $2\ CH_3OH \leftrightarrow CH_3OCH_3 + H_2O$ |
| (4) Overall: | $3\ CO + 3\ H_2 \leftrightarrow CH_3OCH_3$ (DME) $+ CO_2$ |

Two alternative overall reactions, based on reactions (1) to (3), for the synthesis of DME from synthesis gas are:

(5) $2\ CO + 4\ H_2 \leftrightarrow CH_3OCH_3$ (DME) $+ H_2O$

(6) $2\ CO_2 + 6\ H_2 \leftrightarrow CH_3OCH_3$ (DME) $+ 3\ H_2O$

Typically, a direct DME catalyst system contains a methanol synthesis catalyst that is capable of catalysing reactions (1) and (2), and a dehydration catalyst that is capable of catalysing reaction (3), although certain materials are known that are active in all three reactions. These latter materials are also referred to as bifunctional catalysts. For optimal carbon selectivity towards DME, it is generally preferable that DME is formed (mostly) via reaction (5) and/or (6). As such, the majority of the carbon atoms in the reactants end up in the desired product, i.e. DME, and not in a by-product such as COz.

The interplay between the various reactions that together form the DME synthesis, such as between reactions (1) - (3) to give any one of overall reactions (4) - (6), is crucial for satisfactory DME yields, especially since all are equilibrium reactions. The CO to COz molar ratio - or in the context of the present invention the value of x in $CO_x$ - plays a major role. Typically, small amounts of COz can be tolerated in the feedstock. However, since the removal of water by reaction (2) is crucial - after all, water is formed in both reaction (1) and (3) and is a by-product in overall reactions (5) and (6) - the COz content in the feedstock should be kept as low as possible. The presence of COz in the feedstock favours the reverse water gas shift reaction and thus shifts the equilibrium of reactions (1) and (3) towards the reactants. But even with pure CO as carbon oxide species, currently applied methods for the production of DME suffer from the major drawback that DME is formed together with substantial amounts of methanol. The equilibrium mixture typically contains substantial amounts of methanol, which need to be recycled to the catalyst in order to obtain an overall DME yield that is satisfactory. For such a recycle, unreacted starting materials are separated from produced DME and rerouted to the reactor to be contacted again with the catalyst system. Such large recycles hamper the flexibility of the process and increase costs associated with e.g. keeping all reactant streams at the desired temperature and pressure. Moreover, large scale energy-consuming separations (e.g. distillations) are needed to isolated DME from the unreacted reactants and intermediates. Mainly, the separation from MeOH is difficult and costly. Hence, there remains a need in the art to improve the synthesis of DME without the need of extensive separation and major recycles, such that DME production may be more flexible and cost

effective.

Conventional processes for converting syngas into DME are e.g. disclosed in WO 2005/026093, and reviewed by Dieterich (Energy Environ. Sci., 2020, 13, 3207-3252; chapter 3). WO 2005/026093 discloses a process for producing DME wherein COz is converted into CO by a reverse water gas shift reaction, which process requires separation of DME from $CO_2$ ad MeOH as well as a large recycle. The indirect method for the synthesis of DME suffers from the same equilibria and need for recycles, with the only difference that reactions (1) and (2) take place in a different reaction zone as reaction (3). Dieterich explains in section 3.1.1. that the overall reactions for DME require a $H_2$ to CO ratio of at least 1.0 for overall reaction (4), and of at least 2.0 for overall reaction (5). For COz-based DME synthesis, as in overall reaction (6), the optimal $H_2$ to $CO_2$ ratio would be 3.0.

The use of an adsorbent which selectively binds water, to force the equilibrium of reactions (1) and (3) towards the products methanol and DME has been suggested by Iliuta et al. (Chemical Engineering Science 66 (2011) 2241-2251) and Hamidi et al. (Journal of the Taiwan Institute of Chemical Engineers 47 (2015) 105-112). Using a selective water adsorbent in the synthesis of DME is referred to as sorption-enhanced DME synthesis (SEDMES). Iliuta describes a model study for the use of a catalyst system comprising unspecified catalytic particles and zeolite-4A as a third active material. Hamidi also reports a theoretical study, wherein a fixed bifunctional catalyst is combined with flowing zeolite-4A particles which are capable of adsorbing water. Ressler et al. (in Integrated Reaction and Separation Operations, Modelling and experimental validation, editors: Henner Schmidt-Traub and Andrzej Gorak, Springer Verlag, 2006, ISBN10 3-540-30148-8, Chapter 4: *Reactive gas adsorption*) discloses a single-reactor DME synthesis starting from synthesis gas, using a 25:25:50 (by volume) combination of a methanol synthesis catalyst ($Cu/ZnO/Al_2O_3$), gamma-alumina and zeolite-3A. Herein, the catalyst system contains three distinct active materials, wherein the zeolite (3A or 4A) is used as a water adsorbent. SEDMES processes are also disclosed in WO 2017/121817, Van Kampen et al. (Front. Chem. Eng. (2023), 5:1055896) and Skorikova et al. (Front. Chem. Eng. (2020) 2:594884). WO 2017/121817 discloses that super-stoichiometric amounts of $H_2$ are preferred for the synthesis of DME, but stoichiometric and slightly sub-stoichiometric amounts may be suitable. This is reflected in $H_2$ to $CO_x$ ratio of at least x + 0.8, wherein x is in the range of 1 - 2. WO 2017/121817 confirms the stoichiometry requirements disclosed in Dieterich also in the context of SEDMES. Van Kampen discloses experimental validation of the SEDMES technology on a multi-column test-rig under industrially relevant conditions, using stoichiometric amounts of Hz. Skorikova provides a techno-economic evaluation of the SEDMES technology, also using stoichiometric amounts of $H_2$.

The present invention is concerned with providing a process for the production of dimethyl ether in a more efficient and cost-effective way. Most importantly, the need for DME/MeOH separation and MeOH recycles is obviated. Also, the overall yield of DME is improved, in view of less MeOH formation.

**Summary of the invention**

The inventors found that by operating a separation-enhanced DME synthesis with sub-stoichiometric amounts of $H_2$ in the feed gave a product mixture with increased amounts of DME over MeOH. Since less $H_2$ is present in the feed, the carbon selectivity towards the products MeOH and DME overall is reduced (less carbon atoms are converted in a single pass through the reactor), but the formation of MeOH was reduced to a significantly greater extent than the formation of DME. Also, more starting material, CO and/or $CO_2$, remains unconverted and is present in the product mixture, but as these gases are easily separated and recycled to the DME synthesis step for a further pass through the reaction, these carbon atoms are still converted into products and not lost. Hence, with the process according to the invention, the amount of methanol formed is greatly reduced or even eliminated, while the amount DME is not. This improved the overall yield of DME formed and reduces the extent of difficult downstream separation steps to purify the DME.

The process according to the invention is for the synthesis of dimethyl ether (DME) from a feedstock comprising $H_2$ and $CO_x$, and comprises a step (a) wherein the feedstock is subjecting to separation-enhanced DME synthesis by contacting it with one or more catalysts together capable of converting synthesis gas to DME, to obtain a product mixture comprising DME. In the feedstock, x is in the range of 1.0 - 2.0, and the stoichiometry factor *M* is in the range of 0.05 - 1.7. Conventional processes for the synthesis of DME operate with a stoichiometry factor *M* of 2.0, to ensure that most of the carbon atoms are converted into products.

Since the key aspect of the invention is the finding that sub-stoichiometric amounts of $H_2$ are particularly useful in the synthesis of DME, the present invention also concerns the use of a feedstock comprising $H_2$ and $CO_x$, wherein x is in the range of 1.0 - 2.0, and the stoichiometry factor *M* is in the range of 0.8 - 1.7, for the synthesis of DME in a separation-enhanced DME synthesis process. Further effects associated with the use and the process according to the invention include reducing the formation of MeOH during the synthesis of DME, reducing the need for separating DME from MeOH, and reducing MeOH recycles.

## Detailed description

The present invention is based on the finding of the inventors that using sub-stoichiometric amounts of $H_2$ in the synthesis of DME reduced the formation of the undesirable by-product MeOH. Hence, the invention ensures that the overall yield of DME is increased, the formation of MeOH is supressed and the difficult separation of DME and MeOH is greatly reduced or even completely avoided. In the context of the present invention, sub-stoichiometric amounts of $H_2$ is defined as the stoichiometry factor *M* in the range of 0.05 - 1.7, wherein *M* is defined as:

$$M = \frac{[H_2] - [CO_2]}{[CO] + [CO_2]}$$

In a first aspect, the present invention concerns a process for the synthesis of dimethyl ether (DME) from a feedstock comprising $H_2$ and $CO_x$, wherein the process comprises:

(a) subjecting the feedstock to separation-enhanced DME synthesis by contacting it with one or more catalysts together capable of converting synthesis gas to DME, to obtain a product mixture comprising DME;

The feedstock of step (a) has x in the range of 1.0 - 2.0 and the stoichiometry factor *M* in the range of 0.05 - 1.7.

In a second aspect, the invention concerns the use of a feedstock comprising $H_2$ and $CO_x$, wherein x is in the range of 1.0 - 2.0, and the stoichiometry factor *M* is in the range of 0.05 - 1.7, for the synthesis of DME in a separation-enhanced DME synthesis process. Further effects of the use according to the invention include (i) reducing the formation of MeOH during the synthesis of DME; (ii) increasing the overall yield of DME; and (iii) reducing the need for DME/MeOH separation during the synthesis of DME.

Everything defined herein for the process according to the invention equally applies to the use according to the invention, and everything defined herein for the use according to the invention equally applies to the process according to the invention.

*Feedstock*

The feedstock of the process according to the invention comprises $H_2$ and $CO_x$, the reactive species in the synthesis of DME. The term feedstock refers to the gaseous mixture that is subjected to step (a), irrespective of any upstream process steps. Thus, the incoming gaseous mixture may be subjected to further steps upstream of step (a), for example a cleaning step and/or a reverse water gas shift reaction, before it forms the feedstock that is subjected to step (a). In one embodiment, the incoming gaseous mixture is combined with a recycle comprising at least one of CO, $CO_2$ and $H_2$, which typically is obtained as low boiler fraction in step (b) of the process according to the invention.

$CO_x$ denotes CO, $CO_2$ or mixtures thereof, and may also be referred to as "carbon oxide", with CO and $CO_2$ being the two carbon oxide species, or as "CO and/or $CO_2$". The value of x denotes the number of oxygen atoms present per carbon atom in the carbon oxide fraction (i.e. CO + $CO_2$) of the feedstock, irrespective of any further oxygen and/or carbon atoms that may be present in the feedstock. Thus, x is in the range of 1 - 2, wherein x = 1 indicates pure CO and x = 2 indicates pure $CO_2$. An intermediate value for x indicates that a mixture of CO and $CO_2$ is present in the feedstock, which can readily be determined by the skilled person. As example, when x = 1.9, 1.9 oxygen atoms are present per carbon atom, meaning that the molar ratio of CO/$CO_2$ is 1/9. Likewise, when x = 1.5, the CO/$CO_2$ molar ratio is 1/1.

The process according to the invention operates efficiently using a feedstock wherein x is any value in the range 1 - 2. In one embodiment, the feedstock contains $CO_2$, or in other words x > 1, i.e. $1 < x \leq 2$. Preferably, x > 1.2, more preferably x > 1.5, more preferably x > 1.7. In an alternative embodiment, the feedstock contains CO, or in other words x < 2, i.e. $1 \leq x < 2$. Preferably, $CO_2$ is the major carbon oxide species and x is close to 2, e.g. x = 1.5 - 2.0, preferably x = 1.8 - 2.0, or x = 1.9 - 2.0 or even x is about 2. In case a gas containing substantial amounts of COz is used, the gas may be subjected to a reverse water gas shift reaction prior to being used as feedstock for step (a) of the process according to the invention, to increase the content of CO in the feedstock. However, the inventors found that such a pretreatment to raise the content of CO in the feedstock is not needed. In case a recycle from step (b) is mixed into the feedstock, the exact value of x may change a bit. In a preferred embodiment, the incoming gas stream is pure $CO_2$, i.e. x = 2.0, but the recycle typically contains CO and $CO_2$. Thus, the combined incoming streams used as feedstock for step (a) may contain some CO even if a pure $CO_2$ stream is used as incoming gas stream.

In order to determine the stoichiometry of $H_2$, one molecule of $H_2$ per molecule of $CO_2$ in the feedstock is reserved for converting one oxygen atom of $CO_2$ in $H_2O$. Further, two molecules of $H_2$ per carbon atom in $CO_x$ is needed to convert two molecules of $CO_x$ to one molecule of DME and one (further) molecule of $H_2O$. The stoichiometry factor (*M*) takes into account the one additional molecule of $H_2$ is needed in case $CO_2$ is the carbon oxide species, and is thus defined as

follows:

$$M = \frac{[H_2] - [CO_2]}{[CO] + [CO_2]}$$

Overall reactions (4) and (5) require at least two molecules of $H_2$ per carbon oxide species $CO_x$. Therefore, conventional DME syntheses uses feedstocks with $M \geq 2$. The present invention employs sub-stoichiometric amounts of $H_2$, i.e. where $M$ is at most 1.7. In other words, the stoichiometry factor $M$ is in the range of 0.05 - 1.7, preferably in the range of 0.1 - 1.7, more preferably in the range of 0.5 - 1.7. In a preferred embodiment, $M$ is at least 1.0, such as in the range of 1.1 - 1.7, preferably in the range of 1.3 - 1.6, most preferably $M$ is about 1.5. In an alternative embodiment, $M$ is below 1.0, such as in the range of 0.1 - 0.9, preferably in the range of 0.5 - 0.9. The process according to the invention is workable with such low values for $M$, which completely eliminates the formation of MeOH, yet also lowers the productivity of DME and thus requires a larger $CO_2$ recycle. Since good conversions into DME with significant reduced or even eliminated formation of MeOH are already obtained at $M$ above 1.0, it is preferred that $M$ is above 1.0, such as in the range of 1.0 - 1.7.

The feedstocks for the process according to the present invention may be any suitable feedstock. For example, the feedstock may originate from blast furnace gas (BFG), coke oven gas, an off-gas of a carbon capture process, a natural gas, a synthetic natural gas or from the gasification of coal, biomass and/or waste. The feedstock may be subjected to a reforming step upstream of step (a) to increase the $H_2$ and CO content. If needed, additional $H_2$ can be added to such gases to arrive at the above defined stoichiometry factor. Such tuning of the $H_2$ content based on the $CO_x$ content towards the stoichiometry of the reaction is known in the art. In an especially preferred embodiment, the feedstock originates from a non-fossil source, such as from blast furnace gas (BFG), coke oven gas, a carbon capture processes, synthetic natural gas or the gasification of biomass and/or waste.

The feedstock that is subjected to step (a) may be a mixture of streams, for example the incoming gaseous stream is combined with a recycle from step (b). Also, in case the supply of Hz would be reduced, e.g. when there is a shortage in $H_2$ which may be due to intermittency, the incoming flow rate into the reactor of step (a) can be kept constant, by mixing additional $CO_x$ (typically $CO_2$) with the incoming gas stream comprising $H_2$ and $CO_x$. When the flow rate is kept constant, the reactor can continue to operate at the same feed flow rate. This may lower the value of $M$ somewhat, which would beneficially reduce the formation of MeOH.

*Step (a) - separation-enhanced DME synthesis*

The process according to the invention comprises a step (a), wherein the feedstock is subjected to separation-enhanced DME synthesis. Step (a) involves contacting the feedstock with one or more catalysts together capable of converting synthesis gas to DME. In one embodiment as indicated above, the feedstock comprises the recycle from step (b), which may be combined upstream of step (a) or the recycle and the incoming gas may be introduced separately in step (a).

The one or more catalysts may also be referred to as a catalyst system. Catalyst systems for converting synthesis gas to DME are known in the art, and are typically comprised in a catalyst bed. The catalyst system is capable of converting synthesis gas to DME, and typically contains a direct DME catalyst system. The direct DME catalyst system is typically selected from a bifunctional DME synthesis catalyst or a combination of a methanol synthesis catalyst and a methanol dehydration catalyst. Any direct DME catalyst system known in the art is suitable to be used in this respect. Suitable catalysts include alumina containing copper/zinc oxide based catalysts ($Cu\text{-}ZnO\text{-}Al_2O_3$), but other catalysts are also suitable. Step (a) typically operates at 100 - 500 °C, preferably 200 - 350 °C, and 10 - 200 bar, preferably 20 - 100 bar. In an especially preferred embodiment, the pressure of step (a) is in the range of 40 - 100 bar, more preferably 43 - 70 bar. At such pressures, the formation of MeOH was further reduced while good productivity of DME is achieved. Step (a) may further operate with any feed flow rate, which may for example be in the range of 0.5 - 500 $Nm^3/h$, preferably in the range of 1 - 200 $Nm^3/h$, more preferably in the range of 2 - 100 $Nm^3/h$. In an especially preferred embodiment, step (a) operates at a constant feed flow rate, even when the supply of Hz to step (a) varies. The feed can be supplemented with $CO_x$, typically COz, when less $H_2$ can be supplied, such that the overall feed flow rate remains constant. Supplementing with $CO_x$ would lower the value of $M$, which leads to acceptable productivity of DME and a reduced formation of MeOH. Herein, a constant feed flow rate is defined as having a deviation from the average feed flow rate of at most 5 %, typically even at most 1 %.

Step (a) is performed in separation-enhanced mode, or in other words, step (a) is performed in a separation-enhanced DME synthesis (SEDMES) reaction zone. "Separation-enhanced" refers to the separation of one of the products from the equilibrium mixture, such that the equilibrium shifts towards the products. Thus for the DME synthesis of step (a), $H_2O$ is preferably separated from the equilibrium mixture, as this shifts reactions (1) and (3), or (6) towards the products methanol or DME. Suitable means to accomplish separation-enhancement include the use of selective membranes and selective adsorbents. For example, the DME synthesis of step (a) is performed in sorption-enhanced

mode, wherein $H_2O$ is adsorbed onto a water-selective adsorbent. Alternative means to accomplish separation-enhancement involves the use of selective membranes, which are permeable for one of the products, preferably for HzO. When $H_2O$ selectively permeates such a membrane, it is separated from the equilibrium mixture which thus shifts towards the products of the DME synthesis reactions. Preferably, step (a) is performed in sorption-enhanced mode and the catalyst system further comprises a water-selective adsorbent.

A SEDMES reaction zone contains a catalyst system comprising the direct DME catalyst system, which is capable of converting synthesis gas into DME, and means for separating water from the reaction mixture, typically in the form of an adsorbent selective for water or a water-selective membrane.

Any water-selective adsorbent known in the art that is capable to adsorb water but that hardly adsorbs DME and preferably also hardly adsorbs methanol is suitable in this respect. It is further preferred that $H_2$ and $CO_x$ are not adsorbed onto the adsorbent. In a preferred embodiment, the water-selective adsorbent is a porous material, preferably having an average pore diameter of at most 4 Å, such that the pores are capable of accommodating water molecules but no DME molecules. Suitable water-selective adsorbents include silica-based adsorbents, alumina-based adsorbents, and mixtures thereof, especially aluminosilicates such as zeolites, alkaline earth metal oxides (e.g. Ca, Mg, Ba), composites of metal halides, sulfates and phosphates confined in porous matrices, such as in aluminosilicates, graphite and clays. The skilled person finds further guidance in Yuan et al. (Renewable and Sustainable Energy Reviews, 2016, 54, 761-776) and Van Kampen et al. (Front. Chem. Eng., 2023, 5:1055896). In an especially preferred embodiment, the water-selective adsorbent is a zeolite, most preferably a zeolite having an average pore diameter of at most 4 Å. Such materials are selective for water adsorption. Preferably, the average pore diameter is in the range of 1 - 3 Å. Suitable zeolites are type A zeolites, preferably zeolite 3A, zeolite 4A and mixtures thereof. Zeolite 3A, zeolite 4A and mixtures thereof are especially preferred, since they are highly selective for the adsorption of water. When a water adsorbent with larger pores is used, such as zeolite ZSM-5, the selectivity for water adsorption decreases and other molecules such as methanol and DME may be adsorbed onto the adsorbent. Indeed, ZSM-5 does not exhibit sorption-enhancing capacity. As such, the DME yield of the process is decreased and the composition of adsorbed species is less uniform which hampers regeneration of the catalyst system. As will be understood by the skilled person, the catalyst system may contain further components that are inert in or that promote the production of DME at the process conditions, such as a carrier.

Any type of membrane that is known in the art to be selective for permeation of water molecules, while retaining the other gaseous molecules is suitable to be used in a SEDMES reaction zone. The skilled person may find guidance in e.g. Smitha et al., J. Membr. Sci. 2004, 241, 1, 1-21. Suitable membranes include polymeric membranes such as polyvinyl alcohol-based polymeric membranes, polyimide-based membranes and sulfonated polymeric membranes (e.g. SPEEK or nafion-based), zeolite membranes like zeolite A, ZSM-5 and mordenite, silicalite, (amorphous) silica membranes and organic-inorganic hybrid silica membranes. Organic-inorganic hybrid silica membranes include HybSi membranes, known from e.g. Castricum et al. J. Mater. Chem. 2008, 18, 2150-2158 and Agirre Arisketa et al. Sep. Purif. Technol. 2014, 121, 2-12. The SEDMES reactor preferably employs an organic-inorganic hybrid silica membrane, known from e.g. WO 2007/081212, WO 2010/008283, WO 2013/066184 and WO 2014/025259, all of which are herein incorporated by reference in their entirety, and these membranes may be prepared as described therein. Such membranes are resistant to high temperatures and transmembrane pressures, and are thus especially suitable to be used. Higher temperatures and transmembrane pressures lead to larger fluxes which in turn improve the (cost) efficiency of the process according to the invention.

The catalyst system comprised in the SEDMES reaction zone is capable of catalysing reactions (1) - (3) as defined above and at the same time capable of adsorbing water. In view thereof, the equilibrium of reaction (2) is pushed towards the reactants CO and HzO, which in turns enables the use of (substantial amounts of) $CO_2$ as carbon oxide species in the feedstock. Secondly, the equilibria of reactions (1) and (3) are pushed towards the products methanol and DME respectively. The COz produced is converted into CO (and HzO, which is adsorbed) by reaction (2). These effects of the water adsorbent effectuate that the overall "once through" yield of DME is greatly increased, meaning that the need of recycles is reduced. Without being bound to a theory, it is believe that the use of sub-stoichiometric amounts of $H_2$ shifts the equilibrium of reaction (1) slightly towards the reactants, while the majority of the MeOH formed is still converted to DME via reaction (3). Surprisingly, sub-stoichiometric amounts of $H_2$ lead to reduced MeOH formation, but not at the expense of DME. Instead, the relative lack of $H_2$ results in some unconverted carbon, which surprisingly remains as $CO_x$ in the product mixture, and to a lesser extent as MeOH. Moreover, the inventors found that the use of sub-stoichiometric amounts of $H_2$ favours the formation CO over $CO_2$, which reduces cumbersome separation between $CO_2$ and DME from the equilibrium mixture and the need for a $CO_2$ recycle. The product mixture emerging from the SEDMES reaction zone contains mainly DME and some remaining $CO_x$. Even though the $CO_x$ is preferably recycled to the SEDMES reaction zone, this recycle mainly contains CO which is readily separated from DME and used again in a further cycle of step (a). Importantly, the formation of MeOH is generally considered a loss (a waste product), whereas $CO_x$ is not, as that is readily recycled to the DME synthesis reactor. Moreover, separation of MeOH from DME is a difficult and energy-consuming step in conventional sorption-enhanced DME synthesis, and this step can be eliminated in the process according to the invention. Thus, in a preferred embodiment, the process according to the invention does not contain a step wherein MeOH is separated from

DME.

The product mixture of step (a) is optionally further purified via steps (b) and/or (c) as defined below. Step (b) may afford a low boiling fraction comprising one or more of CO, $CO_2$ and $H_2$, preferably it comprises at least $CO_x$, which is preferably recycled to step (a). The recycle comprising $CO_x$, which is typically obtained from step (b), is part of the feedstock of step (a). In other words, the recycle comprising $CO_x$ is combined with an incoming gaseous mixture, which together from the feedstock of step (a). The composition of the feedstock of step (a) is crucial in the present invention, and this feedstock may be obtained from a combination of source, e.g. an incoming gaseous mixture and the recycle.

Step (a) affords an equilibrium mixture that contains at least DME and $CO_x$, the two products of the overall DME synthesis reaction, and some remaining Hz may also be present in the equilibrium mixture as well as some methanol, any inert gaseous species that originate from the feedstock. In a preferred embodiment, DME is isolated from the product mixture, preferably by steps (b) and optionally (c) as defined below. The skilled person finds further guidance for the isolation of DME in Skorikova et al. (Front. Chem. Eng. (2020) 2:594884).

Step (a) further affords a sorbent loaded with water molecules or a membrane loaded with water molecules. Loaded membranes can be regenerated simultaneously to the operation of step (a), by removing water molecules from the permeate side of the membrane. The removal of water from the permeate side is referred to as regeneration step (d). Sorbents are typically used for the operation of step (a) until they are saturated with water, or close to saturation. The loaded sorbent may then be regenerated in a separate regeneration step (d).

*Step (b)*

In a preferred embodiment, the process according to the invention comprises a step (b) wherein the product mixture obtained in step (a) is subjected to a step to separate a low boiling fraction comprising one or more of CO, $CO_2$ and $H_2$, and a high boiling fraction comprising DME. The low boiling fraction is preferably recycled to step (a). Separation of DME from synthesis gas, as well as recycles pf synthesis gas to the DME reactor are well-known in the art, and can be implemented in the present invention in any suitable way. Any means known in the art to separate DME from the product mixture containing DME and synthesis is gas is suitable to be used as step (b). Step (b) is typically performed by flash evaporation, distillation or a combination thereof. Preferably, a flash evaporation is employed in step (b), as that efficiently separates volatile synthesis gas from less volatile DME.

Step (b) affords a high boiling fraction comprising DME. Typically, this high boiling fraction is substantially pure in DME and does not need further purification. In view of the separation-enhanced nature of the DME synthesis of step (a), hardly any water will be present in the high boiling fraction of step (b). Further, in view of the sub-stoichiometric amounts of $H_2$ used in step (a), hardly any MeOH will be present in the high boiling fraction of step (b). In view of the substantial absence of these components, the process according to the present invention is an improvement over conventional DME synthesis, as purification of the DME stream is greatly facilitated. The obtained fraction of DME is the key product of the process according to the invention and can be used as deemed fit. In some embodiments, for example when extremely pure DME is needed, the high boiling fraction obtained in step (b) is further purified in step (c).

Step (b) further affords a low boiling fraction comprising one or more of CO, $CO_2$ and $H_2$. This mixture contains, typically consists essentially of, $H_2$ and $CO_x$, and optionally (inert) permanent gases that are present in the product mixture originating from step (a). Herein, x is typically 1 - 1.4, preferably 1 - 1.1, meaning that the $CO_2$ content is very low to negligible. In view thereof, the separation of step (b) is facilitated and no conversion of $CO_2$ to CO prior to step (a) is needed.

*Step (c)*

In the process according to the invention, step (c) is optional and only preformed if needed. Thus, in one embodiment, the process according to the invention comprises a step (c) wherein the high boiling fraction obtained in step (b) is subjected to a step to separate a low boiling fraction comprising DME, and a high boiling fraction comprising HzO. The high boiling fraction obtained in step (c) may further comprises some traces of MeOH that are formed in step (a). The products of step (c) may also be referred to as the second low boiling fraction and the second high boiling fraction. Any means known in the art to separate DME from the high boiling fraction obtained in step (b) is suitable to be used as step (c). Step (c) is typically performed by flash evaporation, distillation or a combination thereof. Preferably, a distillation is employed in step (c).

Step (c) affords a low boiling fraction comprising DME, typically consisting of DME. The obtained fraction of DME is the key product of the process according to the invention and can be used as deemed fit. Step (b) further affords a high boiling fraction comprising $H_2O$ and possibly MeOH. This high boiling fraction may be discharged from the system as a by-product and used as deemed fit, or it may also be recycled to step (a) to be converted into DME. However, in view of the low MeOH content, such a recycle is typically not useful.

*Step (d)*

The process according to the invention preferably further comprises a step (d) of regenerating the catalyst system of the separation-enhanced DME reaction zone employed in step (a). Regeneration of loaded sorbents of membranes during operation is well-known in the art, and can be performed in any suitable way. For example, water molecules may be removed from the membrane used in step (a) by applying a reduced pressure at the permeate side and/or by applying a sweep gas at the permeate side that removes water molecules from the membrane.

In case step (a) is a sorption-enhanced DME synthesis step, the adsorbent will at a certain point in time be fully occupied with adsorbed water molecules, such that no further water adsorption is possible. At this point, the adsorbent is referred to as "loaded" with water molecules. Regeneration of the adsorbent by drying (removal of water) enables reuse of the catalyst system for a further cycle of DME synthesis. The regeneration may be accomplished by any means known in the art for drying an adsorbent material. Suitable means include reducing the total pressure or the partial pressure of steam in the reactor (e.g. pressure swing adsorption (PSA) or vacuum pressure swing adsorption (VPSA)), increasing the temperature (e.g. temperature swing adsorption (TSA)), contacting the loaded adsorbent with a dry gas (e.g. passing a gas through the reactor), or combinations thereof. The dry gas should contain less than 0.1% water, and may comprise nitrogen, noble gases, hydrocarbons and even synthesis gas. Combinations of drying techniques, e.g. depressurisation and heating, may also be used. In this context, it is preferred that at least two SEDMES reactors are placed in parallel for performing step (a) and (d) simultaneously, such that one reactor may be regenerated while another reactor is being used for the production of DME. As such, continuous DME production is possible.

In case the SEDMES catalyst system contains the water-selective adsorbent having the pores as defined above, regeneration is facilitated compared conventional DME production processes. In view of the small pores of the adsorbent, water will selectively adsorb thereon. Regeneration of the adsorbent will thus afford an effluent gas containing substantially pure steam. No further separation steps need to be performed on this effluent gas to extract valuable compounds such as methanol or even DME.

*Use*

Since the key aspect of the present invention is the use of sub-stoichiometric amounts of $H_2$ in the synthesis of DME, the present invention further concerns the use of feedstock comprising such sub-stoichiometric amounts of $H_2$, as well as $CO_x$, wherein x is in the range of 1.0 - 2.0, for the synthesis of DME in a separation-enhanced DME synthesis process. Herein, sub-stoichiometric $H_2$ is defined by the stoichiometry factor *M* being in the range of 0.05 - 1.7, wherein *M* is defined as:

$$M = \frac{[H_2] - [CO_2]}{[CO] + [CO_2]}$$

In one embodiment, the use according to the invention is for one or more of: (i) reducing the formation of MeOH during the synthesis of DME; (ii) increasing the overall yield of DME; and (iii) reducing the need for DME/MeOH separation during the synthesis of DME, preferably for all three of effects (i) - (iii). Herein, increasing the overall yield of DME refers to the total yield of DME from the feedstock, which may involve several passages through the DME reactor. As hardly any carbon atoms are converted into MeOH, the amount of side-products formed is greatly reduced. In a single passage through the reactor, the conversion of $CO_x$ into DME may be equal or even slightly lower than for conventional DME synthesis, the unconverted carbon atoms are predominantly in the form of $CO_x$, which is readily converted into DME in a further passage through the reactor.

**Description of the figures**

Figure 1 shows the results of the example, obtained at 35 bar. Product selectivities to CO (Fig. 1A), $CO_2$ (Fig. 1B) and MeOH (Fig. 1C) are plotted for a given DME selectivity, for feeds having a stoichiometry factor *M* of 2.0 (white circles) or 1.7 (black circles).

Figure 2 shows the results of the example, obtained at 50 bar. Product selectivities to CO (Fig. 2A), COz (Fig. 2B) and MeOH (Fig. 2C) are plotted for a given DME selectivity, for feeds having a stoichiometry factor *M* of 2.0 (white circles), 1.8 (black circles) or 1.5 (grey circles).

Figure 3 shows the results of the example, obtained at 35 bar. Product selectivity towards MeOH (Fig. 3A) and DME productivity (Fig. 3B) is plotted against cycle duration, for feeds having a stoichiometry factor *M* of 2.0 (white circles) or

1.7 (black circles).
Figure 4 shows the results of the example, obtained at 50 bar. Product selectivity towards MeOH (Fig. 4A) and DME productivity (Fig. 4B) is plotted against cycle duration, for feeds having a stoichiometry factor *M* of 2.0 (white circles), 1.8 (black circles) or 1.5 (grey circles).

## Examples

The results described below have been obtained through modelling using SEDMES simulation code, based on a 1-dimensional pseudohomogeneous reactor model for each of the columns, iterative simulation of each of the columns in the system until cyclic steady state, and time-averaging of the reaction product flows. The model, and the parameters used therein, are described by Van Kampen et *al.* (see (a) Sorption enhanced dimethyl ether synthesis under industrially relevant conditions: experimental validation of pressure swing regeneration, in Reaction Chemistry & Engineering, 2021, 6(2), 244-257; and (b) Sorption enhanced dimethyl ether synthesis for high efficiency carbon conversion: Modelling and cycle design, in Journal of CO2 Utilization, 2020, 37, 295-308). The model used is described in detail in Van Kampen et al. (Continuous multi-column sorption-enhanced dimethyl ether synthesis (SEDMES): Dynamic operation, in Front. Chem. Eng. 2023, 5:1055896, see section 2.4 which is incorporated herein by reference).

Feed mixtures with varying values of *M*, with *M* = 1.5, 1.7, 1.8 or 2.0, are subjected to SEDMES at 35 bar or 50 bar at various cycle durations and a feed flow of 20 $Nm^3/h$. The composition of the feed mixtures is provided in the table below. The obtained product mixtures, based on a given carbon selectivity towards DME, of a single pass through the reactor are given in Figures 1 - 2.

| | Feed 1 | | Feed 2 | | Feed 3 | | Feed 4 | |
|---|---|---|---|---|---|---|---|---|
| | wt% | mol% | wt% | mol% | wt% | mol% | wt% | mol% |
| $[H_2]$ | 12.1 | 75.0 | 11.4 | 73.7 | 11.0 | 73.0 | 10.3 | 71.4 |
| $[CO_2]$ | 87.9 | 25.0 | 88.6 | 26.3 | 89.0 | 27.0 | 89.7 | 28.6 |
| $[CO]$ | 0.0 | 0.0 | 0.0 | 0.0 | 0.0 | 0.0 | 0.0 | 0.0 |
| *M* | 2.0 | | 1.8 | | 1.7 | | 1.5 | |

Figure 1 gives the product selectivities of the single-pass DME synthesis reaction at 35 bar for feed 1 (*M* = 2.0) and feed 3 (*M* = 1.7). The reaction with feed 3 gives similar amounts of COz (Fig. 1B), significantly more CO (Fig. 1A) and significantly less MeOH (Fig. 1C) at a given DME selectivity than the reactions with feed 1. Figure 2 gives the product selectivities of the single-pass DME synthesis reaction at 50 bar for feed 1 (*M* = 2.0), feed 2 (*M* = 1.8) and feed 4 (*M* = 1.5). The reaction with feed 4 gives similar amounts of $CO_2$ (Fig. 2B) significantly more CO (Fig. 2A) and significantly less MeOH (Fig. 2C) at a given DME selectivity than the reactions with feed 1 and feed 2.

The MeOH selectivity is plotted against the cycle duration for feed 1 (*M* = 2.0) and feed 3 (*M* = 1.7) at 35 bar (Fig. 3A). At the same time, the productivity of DME of a single pass through the reactor drops slightly (Fig. 3B), yet since carbon atoms end up as $CO_x$, recycling those to the DME synthesis reactor would convert these into DME. Similar conclusions can be drawn for the reaction at 50 bar, when comparing feed 1 (*M*= 2.0) and feed 4 (*M* = 1.5) (Fig. 4A and 4B). Upon lowering the stoichiometry factor *M*, the selectivity towards MeOH drops, at a constant cycle duration. Hence, overall less MeOH is formed when the feed has a lower value of *M*.

From these results, it can be concluded that lowering the stoichiometry ratio M from 2.0 to 1.5 gives product mixture with a much greater fraction of DME over MeOH. The DME to MeOH ratio increases from about 10 (for *M* = 2.0) to 36 (for *M* = 1.5) at 35 bar. The reaction at 50 bar gives an even greater increase in the DME to MeOH ratio. With that much less MeOH present in the product mixture of the SEDMES step, the downstream separation and purification of DME is much easier, leading to a less energy-consuming and more economical and cost-effective process for the production of DME.

## Claims

**1.** A process for the synthesis of dimethyl ether (DME) from a feedstock comprising $H_2$ and $CO_x$, comprising:

(a) subjecting the feedstock to separation-enhanced DME synthesis by contacting it with one or more catalysts together capable of converting synthesis gas to DME, to obtain a product mixture comprising DME;

wherein in the feedstock x = 1.0 - 2.0, and the stoichiometry factor *M* is in the range of 0.05 - 1.7, wherein *M* is defined as:

$$M = \frac{[H_2] - [CO_2]}{[CO] + [CO_2]}$$

2. The process according to claim 1, wherein step (a) operates at a pressure in the range of 10 - 100 bar(a), preferably in the range of 25 - 45 bar(a).

3. The process according to claim 1 or 2, wherein *M* is in the range of 1.1 - 1.7, preferably in the range of 1.3 - 1.6.

4. The process according to any one of the preceding claims, wherein x is in the range of 1.5 - 2.0, preferably in the range of 1.8 - 2.0.

5. The process according to any one of the preceding claims, wherein the feedstock originates from a blast furnace gas, a coke oven gas, an off-gas of a carbon capture process, a natural gas, a synthetic natural gas or from the gasification of coal, biomass and/or waste.

6. The process according to any one of the preceding claims, further comprising a step of isolating DME from the product mixture, preferably by

    (b) subjecting the product mixture obtained in step (a) to a distillation step to separate a low boiling fraction comprising one or more of CO, COz and $H_2$, and a high boiling fraction comprising DME;
    (c) optionally subjecting the high boiling fraction obtained in step (b) to a second distillation step to separate a low boiling fraction comprising DME, and a high boiling fraction comprising $H_2O$.

7. The process according to claim 6, wherein step (c) is performed and the high boiling fraction obtained in step (c) is not subjected to a third distillation step to separate DME from MeOH.

8. The process according to any one of the preceding claims, wherein the product mixture obtained in step (a), and preferably also the low boiling fraction obtained in step (c), comprises DME and MeOH in a molar ratio DME to MeOH of at least $3.0 \times 10^3$.

9. The process according to any one of the preceding claims, wherein CO and/or COz, preferably the low boiling fraction obtained in step (b), is recycled to the separation-enhanced DME synthesis of step (a), where it is part of the feedstock.

10. The process according to any one of the preceding claims, wherein step (a) is performed in a sorption-enhanced reaction zone, which comprises a direct DME catalyst system and a water-selective adsorbent.

11. The process according to claim 10, wherein the water-selective adsorbent is a zeolite, preferably a zeolite having an average pore diameter of at most 4 Å, and/or wherein the direct DME catalyst system is a bifunctional DME synthesis catalyst or a combination of a methanol synthesis catalyst and a methanol dehydration catalyst.

12. Use of a feedstock comprising $H_2$ and $CO_x$, wherein x is in the range of 1.0 - 2.0, and the stoichiometry factor *M* is in the range of 0.05 - 1.7, wherein *M* is defined as:

$$M = \frac{[H_2] - [CO_2]}{[CO] + [CO_2]}$$

for the synthesis of DME in a separation-enhanced DME synthesis process.

13. Use of a feedstock comprising $H_2$ and $CO_x$, wherein x is in the range of 1.0 - 2.0, and the stoichiometry factor *M* is in the range of 0.05 - 1.7, wherein *M* is defined as:

$$M = \frac{[H_2] - [CO_2]}{[CO] + [CO_2]}$$

for reducing the formation of MeOH during the synthesis of DME in a separation-enhanced DME synthesis process.

*Fig. 1A*

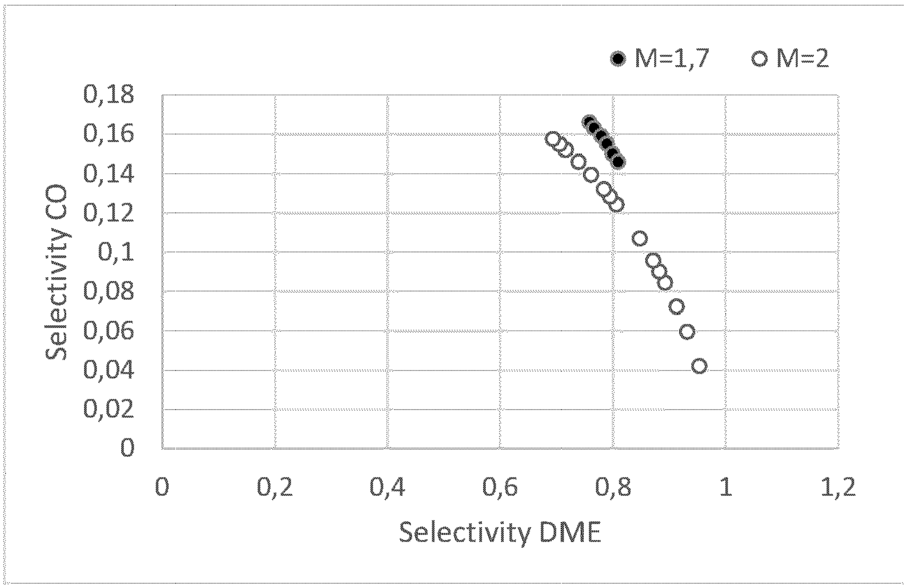


*Fig. 1B*

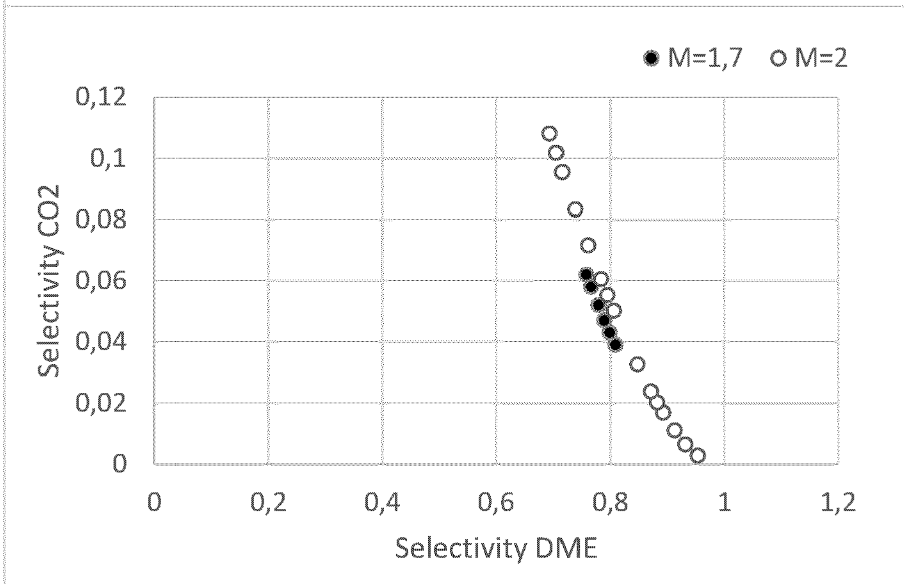


*Fig. 1C*

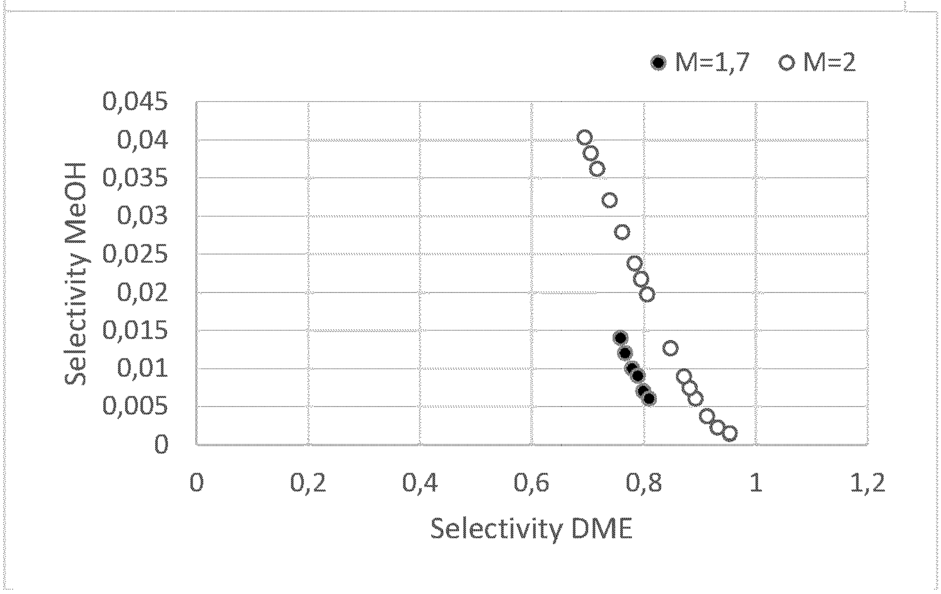

Fig. 2A

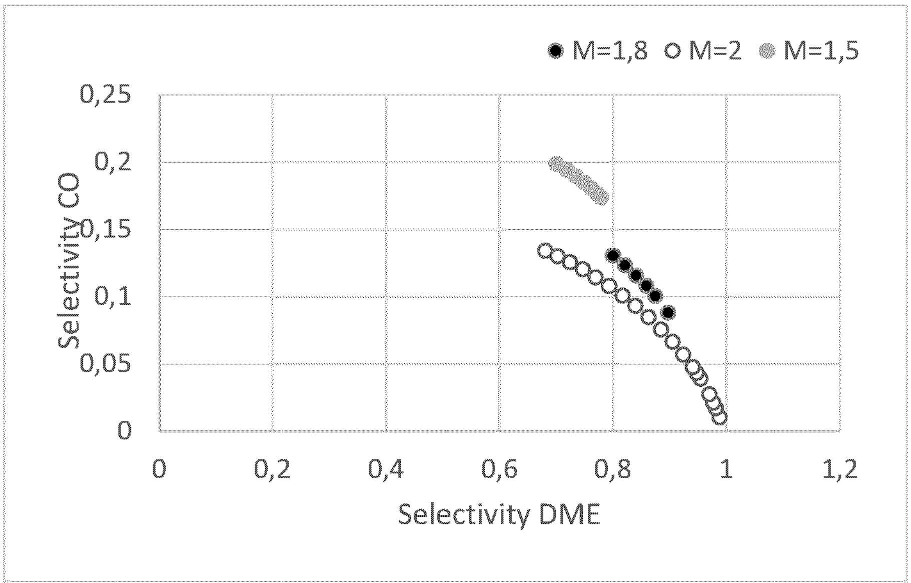


Fig. 2B

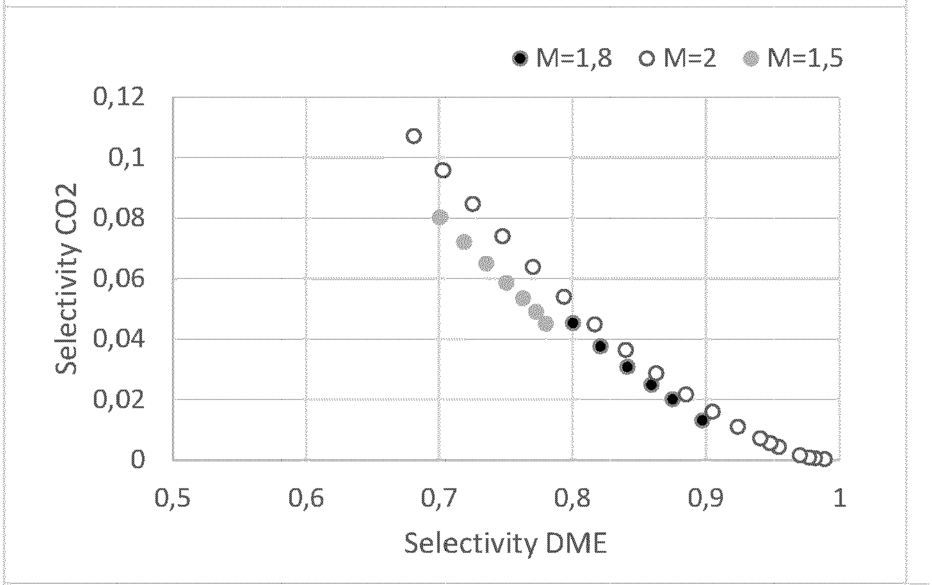


Fig. 2C

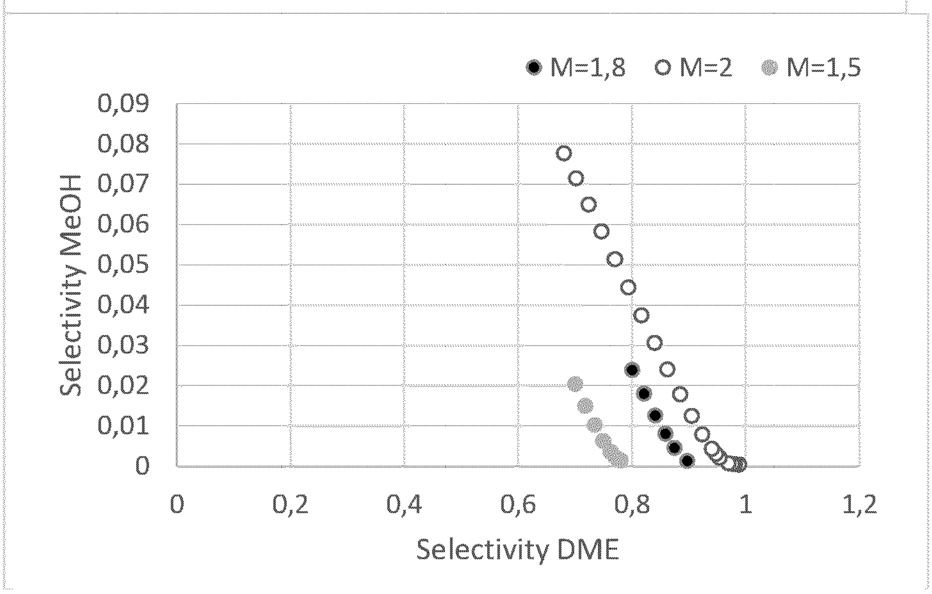

*Fig. 3A*

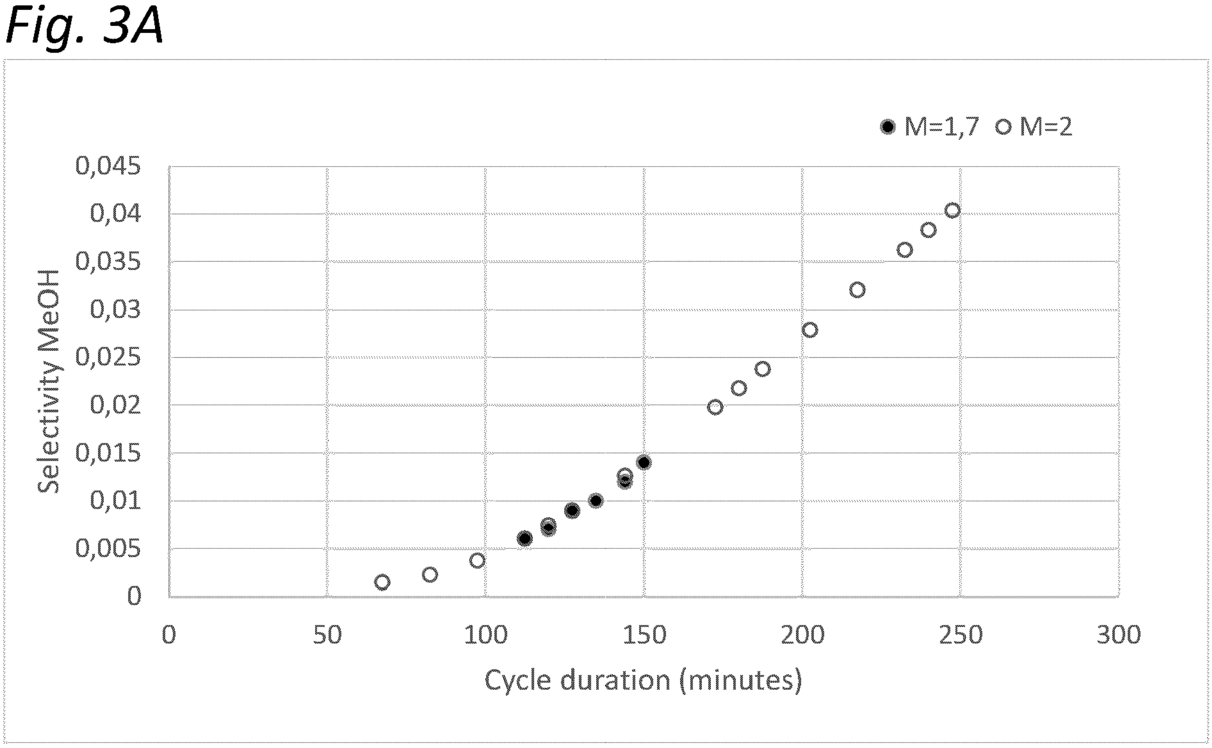
● M=1,7 ○ M=2
Selectivity MeOH
0,045
0,04
0,035
0,03
0,025
0,02
0,015
0,01
0,005
0
0
50
100
150
200
250
300
Cycle duration (minutes)

*Fig. 3B*

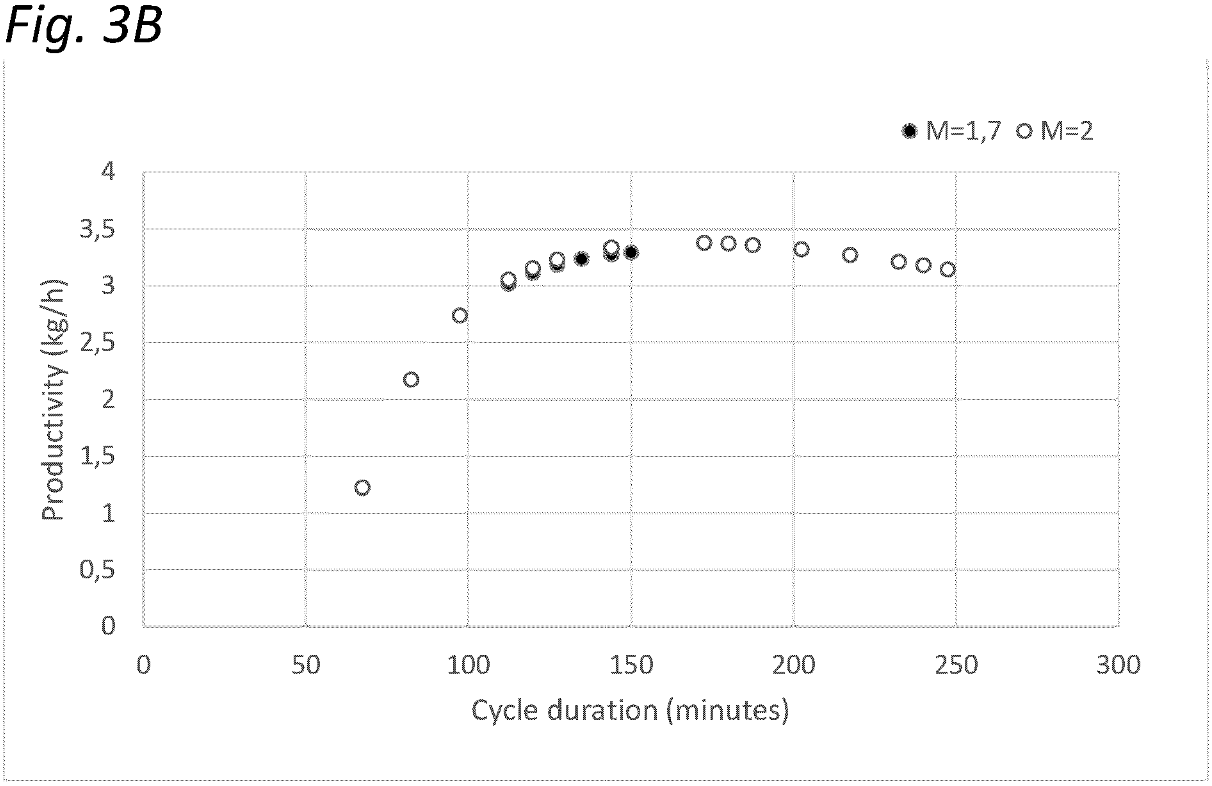
● M=1,7 ○ M=2
Productivity (kg/h)
4
3,5
3
2,5
2
1,5
1
0,5
0
0
50
100
150
200
250
300
Cycle duration (minutes)

*Fig. 4A*

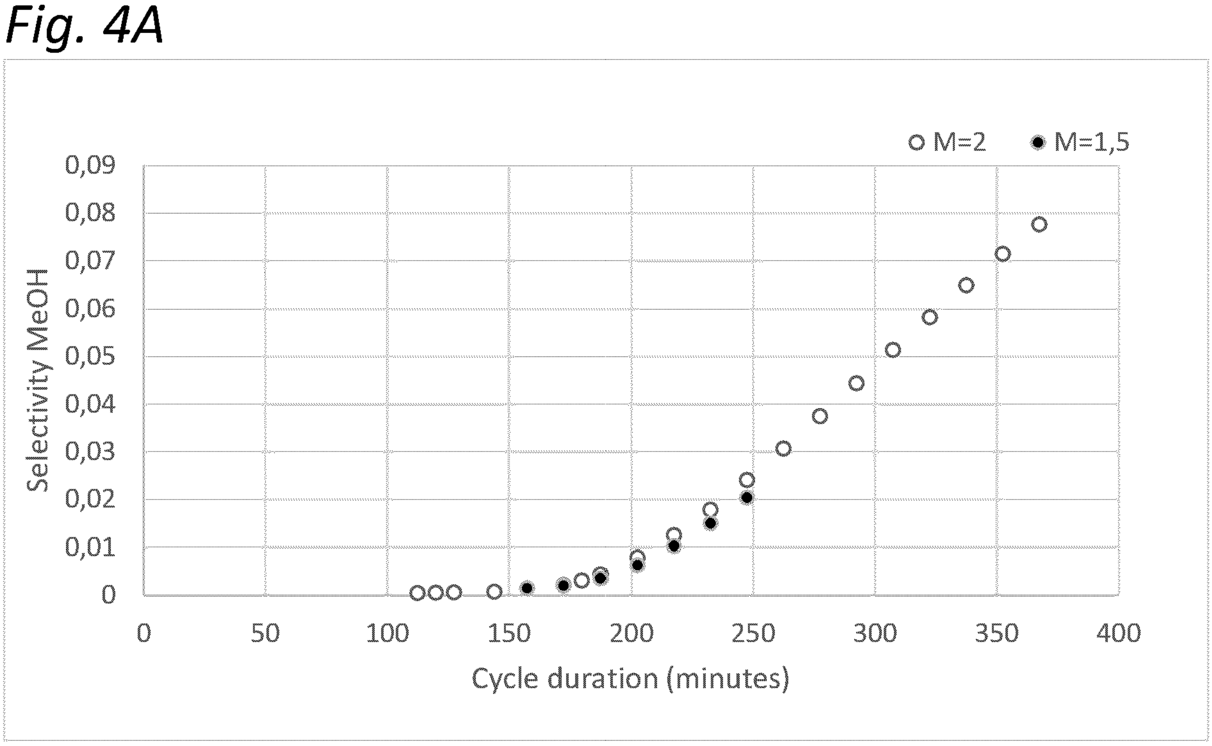
M=2
M=1,5
Selectivity MeOH
0
0,01
0,02
0,03
0,04
0,05
0,06
0,07
0,08
0,09
0
50
100
150
200
250
300
350
400
Cycle duration (minutes)

*Fig. 4B*

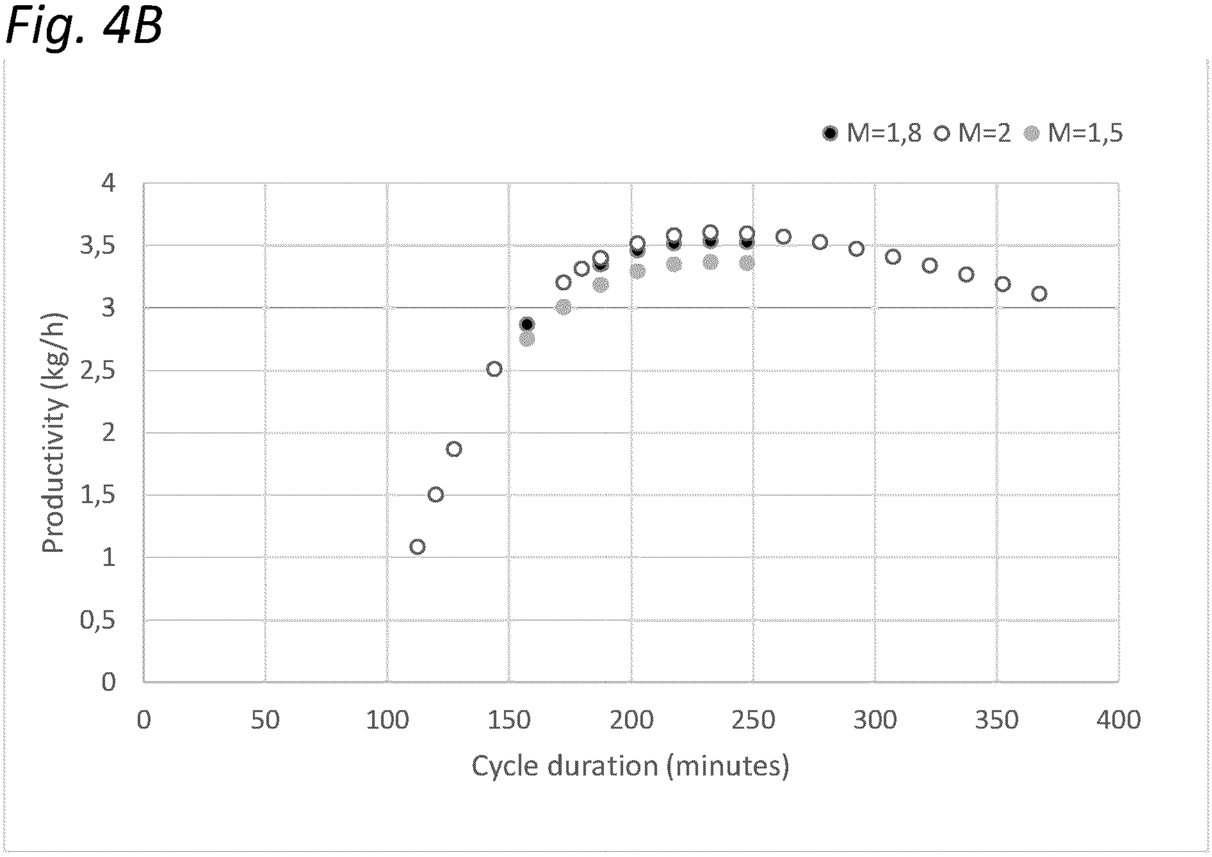
M=1,8
M=2
M=1,5
Productivity (kg/h)
0
0,5
1
1,5
2
2,5
3
3,5
4
0
50
100
150
200
250
300
350
400
Cycle duration (minutes)

Europäisches Patentamt
European Patent Office
Office européen des brevets

# EUROPEAN SEARCH REPORT

Application Number
EP 24 18 8452

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GUFFANTI SIMONE ET AL: "Model Analysis of the Effects of Active Phase Distribution at the Pellet Scale in Catalytic Reactors for the Direct Dimethyl Ether Synthesis", INDUSTRIAL & ENGINEERING CHEMISTRY RESEARCH, [Online] vol. 59, no. 32, 12 August 2020 (2020-08-12), pages 14252-14266, XP093246618, ISSN: 0888-5885, DOI: 10.1021/acs.iecr.0c01938 Retrieved from the Internet: URL:https://pubs.acs.org/doi/pdf/10.1021/acs.iecr.0c01938> [retrieved on 2025-02-04] | 1,2,4,5, 10,12,13 | INV. C07C43/04 C07C41/01 |
| A | * page 14256; tables 1,2 * * results and discussion; page 14256 * * introduction; page 15252 * | 3,6-9,11 | |
| X | CA 2 863 285 A1 (BASF SE [DE]; LINDE AG [DE]) 8 August 2013 (2013-08-08) | 1-5,8, 10-13 | TECHNICAL FIELDS SEARCHED (IPC) C07C |
| Y | * page 4, line 15 - page 6, line 35 * * claims 7,13 * | 6,7,9 | |
| Y | WO 2017/121817 A1 (STICHTING ENERGIEONDERZOEK CENTRUM NEDERLAND [NL]) 20 July 2017 (2017-07-20) * claims 1-14 * * page 4, paragraph [0009] * * page 5, paragraph [0012] * * page 10, paragraph [0026] - page 19, paragraph [0045] * | 6,7,9 | |

The present search report has been drawn up for all claims

| Place of search | Date of completion of the search | Examiner |
|---|---|---|
| The Hague | 17 February 2025 | Seitner, Irmgard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

2

EPO FORM 1503 03.82 (P04C01)

# ANNEX TO THE EUROPEAN SEARCH REPORT ON EUROPEAN PATENT APPLICATION NO. EP 24 18 8452

This annex lists the patent family members relating to the patent documents cited in the above-mentioned European search report. The members are as contained in the European Patent Office EDP file on
The European Patent Office is in no way liable for these particulars which are merely given for the purpose of information.

17-02-2025

| Patent document cited in search report | Publication date | Patent family member(s) | Publication date |
|---|---|---|---|
| CA 2863285 A1 | 08-08-2013 | CA 2863285 A1 | 08-08-2013 |
| | | CN 104203885 A | 10-12-2014 |
| | | EP 2809638 A1 | 10-12-2014 |
| | | RU 2014135282 A | 20-03-2016 |
| | | WO 2013113754 A1 | 08-08-2013 |
| | | ZA 201406313 B | 27-09-2017 |
| WO 2017121817 A1 | 20-07-2017 | CN 108698816 A | 23-10-2018 |
| | | EP 3402746 A1 | 21-11-2018 |
| | | JP 6957475 B2 | 02-11-2021 |
| | | JP 2019501942 A | 24-01-2019 |
| | | KR 20180101523 A | 12-09-2018 |
| | | US 2019016656 A1 | 17-01-2019 |
| | | US 2020399195 A1 | 24-12-2020 |
| | | WO 2017121817 A1 | 20-07-2017 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005026093 A
- WO 2017121817 A, Van Kampen
- WO 2007081212 A
- WO 2010008283 A
- WO 2013066184 A
- WO 2014025259 A


### Non-patent literature cited in the description


- **DIETERICH et al.** *Energy Environ. Sci.*, 2020, vol. 13, 3207-3252
- **DIETERICH**. *Energy Environ. Sci.*, 2020, vol. 13, 3207-3252
- **ILIUTA et al.** *Chemical Engineering Science*, 2011, vol. 66, 2241-2251
- **HAMIDI et al.** *Journal of the Taiwan Institute of Chemical Engineers*, 2015, vol. 47, 105-112
- **RESSLER et al.** Integrated Reaction and Separation Operations, Modelling and experimental validation. Springer Verlag, 2006, vol. 10, 3-540, 30148-8
- *Front. Chem. Eng.*, 2023, vol. 5, 1055896
- **SKORIKOVA et al.** *Front. Chem. Eng.*, 2020, vol. 2, 594884
- **YUAN et al.** *Renewable and Sustainable Energy Reviews*, 2016, vol. 54, 761-776
- **VAN KAMPEN et al.** *Front. Chem. Eng.*, 2023, vol. 5, 1055896
- **SMITHA et al.** *J. Membr. Sci.*, 2004, vol. 241 (1), 1-21
- **CASTRICUM et al.** *J. Mater. Chem.*, 2008, vol. 18, 2150-2158
- **ARISKETA et al.** *Sep. Purif. Technol.*, 2014, vol. 121, 2-12
- *Reaction Chemistry & Engineering*, 2021, vol. 6 (2), 244-257
- *Journal of CO2 Utilization*, 2020, vol. 37, 295-308
- **VAN KAMPEN et al.** Continuous multi-column sorption-enhanced dimethyl ether synthesis (SEDMES): Dynamic operation. *Front. Chem. Eng.*, 2023, vol. 5, 1055896